# EUROPEAN PATENT SPECIFICATION

(11) **EP 3 965 718 B1**
(45) Date of publication and mention of the grant of the patent: **09.07.2025**
(21) Application number: 20802578.3
(22) Date of filing: 05.05.2020
(51) Int. Cl.: A61J 15/00, A61B 17/24, A61M 16/04, A61M 25/01, A61M 5/178

(54) **FEEDING TUBE WITH INTEGRATED STYLET**
ZUFÜHRROHR MIT INTEGRIERTEM FÜHRUNGSSTAB
TUBE D'ALIMENTATION AVEC STYLET INTÉGRÉ

(30) Priority: 06.05.2019 US 201962843673 P
(43) Date of publication of application: 16.03.2022
(73) Proprietor: Bles Biochemicals Incorporated, London, ON N5V 3K4 (CA)
(72) Inventor: NIGH, Harold, London, Ontario N5V 3K4 (CA)
(74) Representative: Zeuner Summerer Stütz
(86) International application number: PCT/CA2020/050599
(87) International publication number: WO 2020/223805

(56) References cited:
- EP-A1- 0 728 496
- EP-B1- 0 810 892
- WO-A1-2013/025993
- WO-A1-2013/177469
- WO-A1-2016/100326
- CA-A1- 2 836 623
- US-A- 4 388 076
- US-A- 4 388 076
- US-A- 4 636 200
- US-A- 4 826 485
- US-A- 5 092 847
- US-A- 5 304 140
- US-A- 5 431 640
- US-A- 5 665 064
- US-A- 6 126 647
- US-A1- 2009 125 002
- US-A1- 2011 137 252
- US-B1- 6 315 789

## Description

### FIELD OF THE INVENTION

The present invention relates to feeding tubes for tracheal insertion and, in particular, to a stylet stiffened feeding tube for use in intratracheal instillation of pulmonary surfactant in a premature infant suffering from Neonatal Respiratory Distress Syndrome (NRDS).

### BACKGROUND

Neonatal Respiratory Distress Syndrome (NRDS) is a respiratory condition affecting premature infants that is treated by insertion of a small diameter catheter into the trachea for intratracheal instillation of pulmonary surfactant, while the infant breathes spontaneously on a continuous positive airway pressure (CPAP) support. Surfactant instillation in spontaneously breathing pre-term infants has the potential to improve their respiratory management. However, endotracheal intubation is recognised as a difficult procedure, even for experienced physicians.

Recently, techniques of minimally or less invasive surfactant therapy (LIST) have been developed, which have been associated with a variety of positive outcomes, including reductions in: early CPAP failure, invasive ventilation requirements, bronchopulmonary dysplasia (BPD), and in the combined outcome of death or BPD.

It has been shown that the catheter used by a physician in performing a LIST procedure affects its effectiveness (see Rigo et al. Devices for less invasive surfactant. Acta Paediatrica 2017; 106: 1091-1096). In particular, a reduction in procedural durations has been shown with stylet-guided catheters, compared to other devices used in LIST procedures, such as handling a feeding tube with Magill forceps or on its own.

However, when using a stylet to stiffen a feeding tube for insertion into the trachea it is important to ensure that the stylet does not extend beyond the end of the feeding tube, as this can result in injury to the patient. For this reason, many stylets have a handle or loop that extends out of the feeding tube adapter and prevents the end of the stylet from protruding from the distal end of the feeding tube. As a result, the stylet must be removed from the feeding tube before attaching a syringe, carrying the pulmonary surfactant, to the feeding tube adapter for the intratracheal instillation.
Document EP 0 728 496 A1 discloses a stylet and a connector therefor. Document US 4 826 485 A discloses a device for guiding tubings. Document US 4 636 200 A is directed to an intubating device. Document US 5 092 847 A discloses an enteral feeding tube stylet. EP 0 810 892 B1 is directed to a stylet device for guiding an enteral feeding tube, and US 4 388 076 A discloses an intubating device.

Accordingly, there is a need for a stylet-stiffened feeding tube for use in LIST procedures that prevents the stylet from protruding from the distal end of the feeding tube, but does not require the stylet to be removed prior to intratracheal instillation of the pulmonary surfactant.

### SUMMARY OF THE INVENTION

A feeding tube with an integrated stylet, according to the present invention, has a flexible feeding tube with distal and proximal ends and an adapter at the proximal end. A stylet with distal and proximal ends is positioned within the feeding tube and has an anchor at the proximal end. The anchor is configured to seat within the adapter to prevent the anchor from entering the feeding tube and has one or more channels extending through the anchor to permit fluid to flow from the adapter through the channels and through the feeding tube with the anchor remaining seated within the adapter.

### BRIEF DESCRIPTION OF THE DRAWINGS

In order that the invention may be more clearly understood, a preferred embodiment thereof will now be described in detail by way of example, with reference to the accompanying drawings, in which:
Figure 1 is a perspective view of a feeding tube with an integrated stylet, according to the present invention, shown attached to a syringe.
Figure 2 is a side view of the device shown in Figure 1.
Figure 3 is a top view of the device, shown in Figure 1.
Figure 4 is a sectional view of the device, along the lines A-A in Figure 3.
Figure 5 is a detail view of the proximal end of the feeding tube, defined by the area B in Figure 4.
Figure 6 is an end view of the anchor of the stylet.
Figure 7 is a sectional view of the anchor, along the lines C-C in Figure 6.

### DESCRIPTION OF THE INVENTION

The feeding tube with an integrated stylet, according to the present invention, prevents the end of the stylet from protruding from the distal end of the feeding tube and permits the stylet to remain in place during surfactant delivery in a LIST procedure.

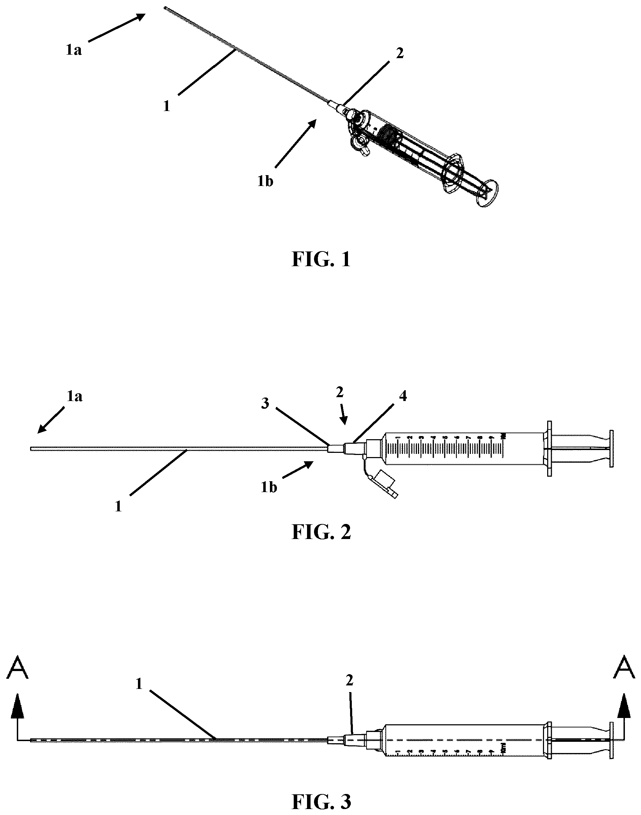

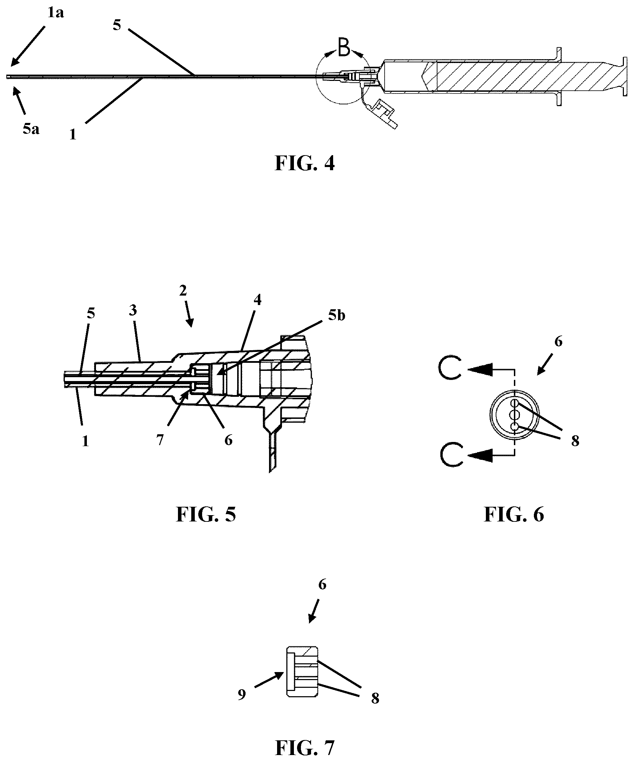

As shown in Figures 1-3, the feeding tube **1** is a flexible tube with distal and proximal ends **1a** and **1b,** having an adapter **2** at its proximal end **1b** for attachment to a syringe tip. The feeding tube **1** may be sized, as required by the particular application. For example, a typical feeding tube **1** for use in a LIST procedure has a length of between 18 cm and 23 cm and an inner diameter of less than 1 mm. Preferably, the feeding tube **1** has a length of 20.5 cm and an inner diameter of 0.91 mm. As shown in Figure 4, the adapter **2** has a root portion **3** that attaches to the proximal end **1b** of the feeding tube **1** and an inlet **4** that extends from the proximal end **1b** of the feeding tube **1.** The interior of the inlet **4** is shaped to engage with the anchor **6** (as described below) and prevent it from entering the feeding tube **1.** Preferably, the interior of the inlet **4** is shaped to form a substantially 90° shoulder **7** adjacent the proximal end **1b** of the feeding tube **1.** Alternatively, the interior of the inlet **4** may be tapered or otherwise shaped such that the inner diameter of the inlet **4** adjacent the perimeter, or a portion of the perimeter, of the proximal end **1b** of the feeding tube **1** is larger than the inner diameter of the feeding tube **1.**

The stylet **5** is a semi-rigid length of wire with distal and proximal ends **5a** and **5b** having an anchor **6** at its proximal end **5b** for seating in the adapter **2.** The stylet **5** is sized to extend substantially the length of the feeding tube **1** for stiffening the feeding tube **1** during insertion into the trachea, but not to protrude from the distal end **1a** of the feeding tube **1.** For example, a typical stylet **5** has a length about 2 cm shorter than the corresponding feeding tube **1,** preferably, about 18.5 cm, and a diameter of between 0.48 mm and 0.54 mm, preferably, 0.51 mm. As shown in Figure 5, the anchor **6** is sized and shaped to seat in the adapter **2** adjacent the proximal end **1b** of the feeding tube **1,** but not to enter the proximal end **1b** of the feeding tube **1.** Preferably, the anchor **6** is generally disc-shaped having a diameter larger than the stylet **5.** For example, a typical anchor **6** has a diameter between 3.15 mm and 3.21 mm, preferably, 3.18 mm and a thickness. The anchor **6** seats against the shoulder **7** formed in the inlet **4** of the adapter **2,** adjacent the proximal end **1b** of the feeding tube **1.**

Where the anchor **6** is generally disc-shaped, the shoulder **7** is generally annular with a diameter slightly larger than the anchor **6.** The anchor **6** thereby seats against the shoulder **7** within the adapter **2** and is prevented from entering the feeding tube **1.** Alternatively, the anchor **6** may have a generally spherical shape, which seats against a correspondingly rounded shoulder **7.** Similarly, the anchor **6** may be dome-shaped, with the rounded side being the distal side of the anchor **6,** which is attached to the proximal end **5b** of the stylet **5.** Various other shapes are possible for the anchor **6,** including a cone-shaped anchor **6** or a triangular, rectangular, pentagonal, hexagonal, etc. prism-shaped anchor **6.** Regardless of the shape of the anchor **6,** the shoulder **7** is shaped complementary to the anchor **6** so that the anchor **6** seats against it, within the adapter **2,** and is thereby prevented from entering the feeding tube **1.**

As shown in Figures 6 and 7, the anchor **6** has one or more channels **8** extending through the thickness of the anchor **6** to permit fluid, such as the pulmonary surfactant from a syringe, to pass through the anchor **6.** The stylet **5** thereby provides added stiffness to the feeding tube **1,** which facilitates insertion into the trachea, while permitting the stylet **5** to remain in place during surfactant delivery. Preferably, as shown in Figure 7, the anchor **6** has two channels **8** on either side of the stylet **5,** with a diameter between 0.45 mm and 0.55 mm, preferably 0.50 mm. Preferably, the anchor **6** also has an annular recess **9** formed about the stylet **5** on the side of the anchor **6** abutting the shoulder **7.** The recess **9** facilitates the flow of fluid from the channels **8** into the annular space surrounding the stylet **5** in the feeding tube **1.** The channels **8** open into the recess **9,** which has a depth sufficient to prevent the shoulder **7** from impeding the flow of fluid through the channels **8.** For example the depth of the recess **9** may be between 0.45 mm and 0.55 mm, preferably, 0.50 mm. In an exemplary embodiment, the diameter of the anchor **6** is 3.18 mm, the length and diameter of the stylet **5** are 18.5 cm and 0.51 mm, respectively, and the diameter of the channels **8** and the depth of the recess **9** are both 0.50 mm.

Preferably, the anchor **6** and the stylet **5** are removable from the feeding tube **1** by unseating the anchor **6** and removing it from the proximal end **1b** of the adapter **1.** Alternatively, the anchor **6** may be permanently attached or formed integrally with the feeding tube **1** or the adapter **2.**

In operation, a physician treating a pre-term infant suffering from NRDS grasps the proximal end **1b** of the feeding tube **1** and/or the adapter **2.** The stylet **5** provides stiffening to the feeding tube **1** to facilitate insertion of the distal end **1a** of the feeding tube **1** by the physician into the trachea of the patient. The physician may then attach a syringe loaded with pulmonary surfactant onto the inlet **4** of the adapter **2** and begin the intratracheal instillation. The pulmonary surfactant is permitted to flow through the channels **8** in the anchor **6** and through the feeding tube **1.** As a result, the present invention eliminates the step of first removing the stylet **5** from the feeding tube **1,** before beginning intratracheal instillation.

Although the present invention has been described with reference to its application in LIST procedures for the treatment of pre-term infants suffering from NRDS, it may applied in other areas and in other treatments where it is desirable to use a stylet-stiffened feeding tube that permits the flow of fluids through the feeding tube, without removal of the stylet.

The present invention has been described and illustrated with reference to an exemplary embodiment, however, it will be understood by those skilled in the art that various changes may be made without departing from the scope of the invention as set out in the following claims. Therefore, it is intended that the invention not be limited to the particular embodiments disclosed herein.

## Claims

1. A feeding tube with an integrated stylet, having a flexible feeding tube (1) with distal and proximal ends and an adapter (2) at the proximal end, and a stylet (5) with distal and proximal ends positioned within the feeding tube (1) having an anchor (6) at the proximal end, **characterized in that** the anchor (6) is configured to seat within the adapter (2) to prevent the anchor (6) from entering the feeding tube (1), and wherein the anchor (6) has one or more channels (8) extending through the anchor (6) to permit fluid to flow from the adapter (2) through the one or more channels (8) and through the feeding tube (1) with the anchor (6) remaining seated within the adapter (2).

2. The feeding tube with an integrated stylet of claim 1, wherein the anchor (6) has opposing proximal and distal sides and a perimeter edge therebetween, and wherein the one or more channels (8) extend between the proximal and distal sides.

3. The feeding tube with an integrated stylet of claim 2, wherein the one or more channels (8) are spaced apart from the perimeter edge.

4. The feeding tube with an integrated stylet of claim 3, wherein the distal side of the anchor (6) is attached to the proximal end of the stylet (5) and has a recess formed thereon in fluid communication with the one or more channels (8).

5. The feeding tube with an integrated stylet of claim 4, wherein the feeding tube (1) has an inner diameter and the stylet (5) has an outer diameter smaller than the inner diameter of the feeding tube (1), thereby providing an annular space surrounding the stylet in the feeding tube.

6. The feeding tube with an integrated stylet of claim 5, wherein the adapter (2) has an inlet extending from the proximal end of the feeding tube (1) and defining a shoulder shaped complementary to the anchor (6) to engage with the anchor and prevent it from entering the feeding tube (1).

7. The feeding tube with an integrated stylet of claim 6, wherein the anchor (6) is generally disc shaped and the shoulder is generally annular.

## Patentansprüche

1. Zuführrohr mit integriertem Führungsstab, umfassend ein flexibles Zuführrohr (1) mit distalem und proximalem Ende sowie einem Adapter (2) am proximalen Ende, und einen innerhalb des Zuführrohrs (1) angeordneten Führungsstab (5) mit distalem und proximalem Ende und einem Anker (6) am proximalen Ende, **dadurch gekennzeichnet, dass** der Anker (6) so ausgestaltet ist, dass er innerhalb des Adapters (2) sitzt und verhindert, dass der Anker (6) in das Zuführrohr (1) eintreten kann, wobei der Anker (6) einen oder mehrere Kanäle (8) aufweist, die sich durch den Anker (6) erstrecken, um das Durchfließen von Fluid vom Adapter (2) durch den oder die Kanäle (8) und durch das Zuführrohr (1) zu ermöglichen, während der Anker (6) innerhalb des Adapters (2) verbleibt.

2. Zuführrohr mit integriertem Führungsstab gemäß Anspruch 1, wobei der Anker (6) gegenüberliegende proximale und distale Seiten sowie eine Umfangskante dazwischen aufweist, und wobei sich der oder die Kanäle (8) zwischen der proximalen und der distalen Seite erstrecken.

3. Zuführrohr mit integriertem Führungsstab gemäß Anspruch 2, wobei der oder die Kanäle (8) mit Abstand zur Umfangskante angeordnet sind.

4. Zuführrohr mit integriertem Führungsstab gemäß Anspruch 3, wobei die distale Seite des Ankers (6) am proximalen Ende des Führungsstabs (5) befestigt ist und eine dort ausgebildete Vertiefung aufweist, die in Fluidverbindung mit dem oder den Kanälen (8) steht.

5. Zuführrohr mit integriertem Führungsstab gemäß Anspruch 4, wobei das Zuführrohr (1) einen Innendurchmesser aufweist und der Führungsstab (5) einen kleineren Außendurchmesser als der Innendurchmesser des Zuführrohrs (1) aufweist, wodurch ein ringförmiger Zwischenraum geschaffen wird, der den Führungsstab im Zuführrohr umgibt.

6. Zuführrohr mit integriertem Führungsstab gemäß Anspruch 5, wobei der Adapter (2) einen Einlass aufweist, der sich vom proximalen Ende des Zuführrohrs (1) erstreckt und eine Schulter definiert, die komplementär zum Anker (6) geformt ist, um mit diesem zusammenzuwirken und zu verhindern, dass er in das Zuführrohr (1) eintritt.

7. Zuführrohr mit integriertem Führungsstab gemäß Anspruch 6, wobei der Anker (6) allgemein scheibenförmig und die Schulter allgemein ringförmig ausgebildet ist.

## Revendications

1. Tube d'alimentation avec stylet intégré, comprenant un tube d'alimentation flexible (1) doté d'extrémités distale et proximale et un adaptateur (2) à l'extrémité proximale, et un stylet (5) positionné à l'intérieur du tube d'alimentation (1), doté d'extrémités distale et proximale ainsi que d'une ancre (6) à l'extrémité proximale, **caractérisé en ce que** l'ancre (6) est conçue pour être logée dans l'adaptateur (2) afin d'empêcher l'entrée de l'ancre (6) dans le tube d'alimentation (1), l'ancre (6) comportant un ou plusieurs canaux (8) traversant l'ancre (6) pour permettre à un fluide de s'écouler depuis l'adaptateur (2), à travers le ou les canaux (8), puis à travers le tube d'alimentation (1), l'ancre (6) restant logée dans l'adaptateur (2).

2. Tube d'alimentation avec stylet intégré selon la revendication 1, dans lequel l'ancre (6) présente des faces proximale et distale opposées et un bord périphérique entre celles-ci, et où le ou les canaux (8) s'étendent entre les faces proximale et distale.

3. Tube d'alimentation avec stylet intégré selon la revendication 2, dans lequel le ou les canaux (8) sont espacés du bord périphérique.

4. Tube d'alimentation avec stylet intégré selon la revendication 3, dans lequel la face distale de l'ancre (6) est fixée à l'extrémité proximale du stylet (5) et présente un évidement en communication fluidique avec le ou les canaux (8).

5. Tube d'alimentation avec stylet intégré selon la revendication 4, dans lequel le tube d'alimentation (1) présente un diamètre intérieur et le stylet (5) présente un diamètre extérieur inférieur au diamètre intérieur du tube d'alimentation (1), créant ainsi un espace annulaire entourant le stylet dans le tube d'alimentation.

6. Tube d'alimentation avec stylet intégré selon la revendication 5, dans lequel l'adaptateur (2) comporte une entrée s'étendant depuis l'extrémité proximale du tube d'alimentation (1) et définissant un épaulement complémentaire à l'ancre (6), afin d'engager l'ancre et empêcher son entrée dans le tube d'alimentation (1).

7. Tube d'alimentation avec stylet intégré selon la revendication 6, dans lequel l'ancre (6) est généralement en forme de disque et l'épaulement est généralement annulaire.
